# EUROPEAN PATENT APPLICATION

(11) **EP 1 842 905 A1**
(43) Date of publication of application: **10.10.2007**
(21) Application number: 06712905.6
(22) Date of filing: 02.02.2006
(51) Int. Cl.: C12N 5/06, A61L 27/00, C12N 15/09

(54) **METHOD OF INDUCING DIFFERENTIATION OF EMBRYO-STEM CELL INTO HEPATOCYTE AND HEPATOCYTE INDUCED BY THE METHOD**

(30) Priority: 03.02.2005 JP 2005028200
(71) Applicant: National University Corporation Okayama University, Okayama-shi, Okayama 700-8530 (JP)
(72) Inventor: TANAKA, Noriaki Graduate School of Med., Dent. & Pharm., Okayama-shi, Okayama 7008558 (JP); KOBAYASHI, Naoya, Cho 2-Chome, Okayama-shi, Okayama 7008558 (JP)
(74) Representative: TBK-Patent
(86) International application number: PCT/JP2006/301762
(87) International publication number: WO 2006/082890

(57) **Abstract**

With respect to a method for differentially inducing embryo-stem cells into hepatocytes, in order to obtain safe hepatocytes that are adequately functionable and able to supply in large quantity, a method for differentially inducing embryo-stem cell into hepatocyte, wherein the embryo-stem cells are cultured in the presence of deletion type hepatocyte growth factor is provided. Further, a method for differentially inducing embryo-stem cells into hepatocytes comprising (a) a step of forming the embryoid body of the embryo-stem cells and (b) a step of culturing the embryoid body in the presence of deletion type hepatocyte growth factor is provided.

## Description

### TECHNICAL FIELD

The present invention relates to a method for differentially inducing embryo-stem cells (hereinafter, also called as ES cells) into hepatocytes and hepatocytes induced thereby, and a bioartificial liver.

### BACKGROUND ART

The liver is the maximum substantial organ in the human body and its functions prevails in various varieties such as bilirubin metabolism, drug metabolism and the production of blood coagulation factor in addition to the metabolism of glucose, protein and fat, and the functions of the liver counts several hundreds including unknown functions; therefore it plays very important roles in organism. Accordingly, serious liver disease is very dangerous for the life of a subject even if it is temporary one.

On the other hand, the liver has active reproducibility and even if a subject is subject to hepatic damage because of fulminant liver failure, the subject recovers if liver function can be replaced for about one week. It is a fact that the implantation of the liver is the most effective for such serious liver disease, but all of the subjects cannot receive its merit considering serious donor deficiency. Although a certain level of critical care rate is obtained by combining continuous filtration dialysis with plasma exchange, it is hardly said to be an adequate treatment, the establishment of more effective therapy is required and needs for the development of artificial liver for remedy have been enhanced. Consequently, therapy expected is a bioartificial liver filled with cells for making the most use of the metabolism ability of alive cell and the synthesis ability of proteins.

The artificial liver can be said as an artificial liver device that incorporates hepatocytes in the carrier to be fixed and to be a reactor and imitates the liver in the human body. The blood in a subject is introduced in the device and the removal of toxin in the blood and the feed of biologically active substances such as coagulation factor derived from the liver cells can be carried out by utilizing the metabolizing ability of hepatocytes. As the source of the cells, healthy human hepatocytes are ideal but it is extremely difficult to obtain them because of the deficiency of the donor liver. In Europe and the U.S.A., the liver incompatible with implantation is leveraged to the separation of the hepatocytes and clinically applied to the implantation of the hepatocytes and the bioartificial liver, but in Japan, the liver incompatible with implantation is prescribed as incineration and it cannot be leveraged to the bioartificial liver.

As a countermeasure for the problem, induction from cells such as human peripheral blood stem cell, marrow stem cell, liver precursor cell to human hepatocyte has been studied, but these cells are deficient in proliferation potency and it is not actual to secure the number of cells (at least 1 × 10⁹ cells) appropriate for application to the bioartificial liver. On the other hand, although the clinical trial of the bioartificial liver remedy using pig hepatocytes for human has been conducted in the USA, Europe and China, the infectious diseases common to human and animal such as swine endogeneous retrovirus infection and hepatitis E are seen as a problem, resulting in difficulty in future development.

Further, the establishment of immortalized cell by genetic recombination setting finally differentiated human cell as a target has been studied, but since foreign gene is introduced in methods of using these genetic recombination, high hurdle in safety that the chromosome of cell introducing gene is changed and the cell is tumorigenically transformed in accordance with the change exists in application for human (Craig D. Woodworth et. al., Molecular and Cellular Biology Oct. 4492-4501, 1988). Further, with respect to the use of retrovirus vector, since an accident that γδ-T cellular leukemia was developed in genetic treatment for a kid subject suffering from X-chromosome sequential congenital and double serious immune deficiency disease (X-Linked SCID) using retrovirus vector was reported in September, 2002, in France, careful posture has been assumed (S. Hacein-Bey-Abina et. al., Science, Vol.302 (17), pp415-419, 2003).

In view of the situation described above, the study of cell source near to more natural form has been required for developing the bioartificial liver that can be realized for critical application.

On the other hand, ES cell 1) can be differentiated to all kinds of cells composing organism, 2) can be cultured in large quantity at low cost because of having the ability of semipermanent proliferation and 3) can apply an induction method using cell growth factor without the genetic recombination; therefore the ES cell has some advantages that cells near to natural posture in comparison with genetic modified cell can be available.

As the method of differentiating and inducing the hepatocyte from the stem cell, factors such as Hepatocyte Growth Factor (HGF), Fibroblast Growth Factor (FGF), dexamethasone, oncostatine M (belongs to the family of interleukin 6), n-butyric acid are used, but induction to adequately functionable hepatocyte is not possible (Rambhatla L et. al., Cell Transplant. Vol.12, pp1-11, 2003, Schwartz RE et. al., J. Clin. Invest. Vol.109, pp 1291-1302, 2002 and Japanese Patent Unexamined Patent Application Publication No. 2003-530879). Further, although there is reported an example that the differentiation induction of the ES cell to the hepatocyte is promoted by transplanting the ES cells to the liver of a mouse inducing liver failure (Yamamoto H et. al., Hepatology 2003, Vol.37, 983, 2003), there are some problems, its operation is troublesome and the differentiated hepatocytes must be collected from the liver of the mouse with impairment.

### DISCLOSURE OF INVENTION

An object of the present invention is to provide safe hepatocytes which are adequately functionable and able to supply in large quantity and use thereof, and in particular, a bioartificial liver using the hepatocytes.

As a result of an intensive study to solve the above problems, we have found that the ES cell can be differentially induced into the hepatocyte efficiently by using deletion type hepatocyte growth factor (hereinafter, also called as dHGF) being the natural variant of HGF, to complete the present invention.

Namely, the present invention provides a method for differentially inducing embryo-stem cells into hepatocytes shown below, the hepatocytes induced thereby, a bioartificial liver using the hepatocytes, a method for testing a drug using the hepatocytes and a process for producing a physiologically active substance using the hepatocytes.
(1) A method for differentially inducing embryo-stem cells into hepatocytes, wherein the embryo-stem cells are cultured in the presence of deletion type hepatocyte growth factor.
(2) A method for differentially inducing embryo-stem cells into hepatocytes comprising the steps of:
   (a) forming an embryoid body of the embryo-stem cells; and
   (b) culturing the embryoid body in the presence of deletion type hepatocyte growth factor.
(3) The method described in the above-mentioned (1) or (2), wherein the embryo hepatocytes are derived from mammal.
(4) The method described in (3), wherein the mammal is human.
(5) The method described in (3), wherein the mammal is mouse.
(6) The method described in any one of (2) to (5), wherein the embryoid body is formed by suspension culture.
(7) The method described in any one of (1) to (5), wherein the culture is carried out by three-dimensional culture.
(8) Hepatocytes induced by the method described in any one of (1) to (7).
(9) A bioartificial liver comprising the hepatocytes described in (8).
(10) A process for producing a bioartificial liver comprising the steps of:
   (a) forming an embryoid body of embryo-stem cells;
   (b) filling the embryoid body obtained in the bioartificial liver reactor; and
   (c) culturing the embryoid body in the presence of deletion type hepatocyte growth factor in the bioartificial liver reactor.
(11) A method for testing a drug using hepatocytes described in (8).
(12) A process for producing a physiologically active substance using hepatocytes described in (8).

### BRIEF DESCRIPTION OF DRAWINGS

FIG. 1 is the photograph of a phase microscope of the mouse hepatocytes (Example 3) differentially induced from the mouse ES cells on a non-woven cloth according to the method of the present invention. A plurality of nuclei 2 that are characteristic of the hepatocyte are observed in the hepatocyte 1 differentially induced.
FIG.2 (a) is the photograph of a scanning electron microscope of the embryoid body 3 just after sowing that adheres on non-woven fiber 4 and FIG.2 (b) is the photograph of a scanning electron microscope of the hepatocyte 1 that adheres on the non-woven fiber 4.

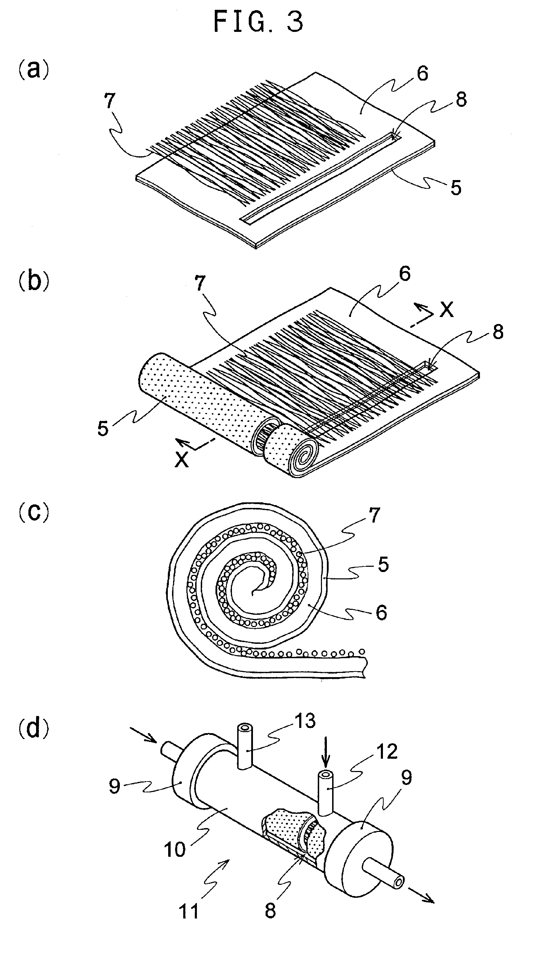

FIG.3 is a diagram showing the one embodiment of production process exemplifying one embodiment of the bioartificial liver reactor of the present invention. FIG.3 (a) is a diagram in which hollow fiber membrane 7 is arranged on the non-woven cloth 6 treated with the backing 5. Hereat, the slit 8 is provided on the non-woven cloth 6 treated with the backing 5. FIG.3 (b) is a diagram showing a process of winding (a) in a roll shape. FIG.3 (c) is a sectional magnified view as taken along line X-X of FIG.3 (b). FIG.3 (d) is a schematic diagram showing a bioartificial liver reactor 11 in which a roll comprising the hollow fiber membrane 7 and the non-woven cloth 6 is assembled in a tubular container 10 on both sides of which a liquid leak-preventing member 9 is provided.
FIG.4 is a photograph showing the expression of hepatic specific gene and GAPDH gene being endogeneous standard in the mouse hepatocytes differentially induced from the mouse ES cells by the method of the present invention. Lanes 1 to 6 show respectively a size marker, the undifferentiated ES cells, feeder cells, the differentially induced ES cells (Example 2), the ES cells differentially induced on a non-woven cloth (Example 3) and the mouse hepatocytes.
FIG.5 is a graph showing the drug metabolic ability of the mouse hepatocytes differentially induced from by the method of the present invention.
FIG.6 is a graph showing the urea production ability of the mouse hepatocytes differentially induced from by the method of the present invention.
FIG.7 is a graph showing the albumin production ability of the mouse hepatocytes differentially induced from by the method of the present invention.
FIG.8 is the photograph of a phase microscope showing embryoid body (Example 5) formed from the human ES cells by the method of the present invention.
FIG.9 is the photograph of a phase microscope of the human hepatocytes differentially induced from the human ES cells by the method of the present invention.
FIG.10(a) is the photograph of a scanning electron microscope of the embryoid body 23 just after sowing that adheres on non-woven fiber 4 (Example 6). FIG. 10 (b) is the photograph of a scanning electron microscope of the hepatocyte 21 (Example 6) differentially induced that adheres on the non-woven fiber 4.

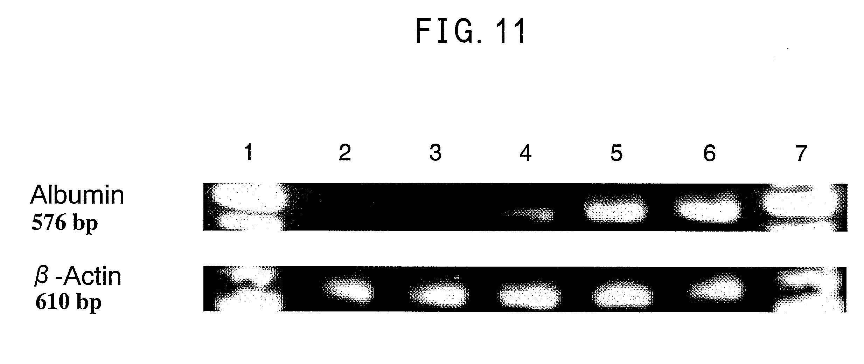

FIG.11 is a photograph showing the expression of albumin gene being the hepatocyte specific gene in the human hepatocytes differentially induced from the human ES cells by the method of the present invention and β-actin gene being endogenous control. Lanes 1 to 7 show respectively a size marker, the undifferentiated human ES cells, the human ES cells forming embryoid body, the human ES cells forming the embryoid body and then differentially induced without using a non-woven cloth (Example 5), the human ES cells forming the embryoid body and then differentially induced on a non-woven cloth (Example 6), the human hepatocytes and a size marker.
FIG. 12 is the photograph of a scanning electron microscope of the hepatocytes differentially induced from the human ES cells on a non-woven cloth by the method of the present invention (Example 6).
FIG. 13 is a graph showing the drug metabolic ability of the mouse hepatocytes differentially induced by the method of the present invention.
FIG. 14 is a graph showing the urea production ability of the human hepatocytes differentially induced by the method of the present invention.
FIG.15 is a graph showing the albumin production ability of the human hepatocytes differentially induced by the method of the present invention.

### BEST MODE FOR CARRYING OUT THE INVENTION

The present invention relates to a method for differentially inducing ES cell into hepatocyte by using dHGF being the natural variant of HGF having less cellular disorder in comparison with HGF.

The method for differentially inducing hepatocyte from ES cell of the present invention comprises (a) a step of forming the embryoid body of the ES cells and (b) a step of culturing the obtained embryoid body in the presence of dHGF.

The ES cell is preferably ES cell derived from a mammal and the mammal includes primates such as human and crab-eating monkey, and mouse.

dHGF is splice variant produced by splicing natural HGF to deleting 5 amino acids (refer to FEBS Letters 434 (1998), pp 165-170). dHGF can be produced according to the descriptions of Japanese Examined Patent Publication No.7-68272 and Japanese Unexamined Patent Publication No.7-188292 that is the divisional application thereof. Further, dHGF is described as TCF-II in these publications.

The ES cell can be produced embryo being the initial stage at which fertile egg continues to grow. The fertile egg prepared by coupling egg cell with sperm on a way to development into fetus repeats scissions to two, four, eight --- and becomes a state called as blastocyst on the 5^{th} or the 6^{th} day. The blastocyst is composed of blastocoel embracing the internal clump of cell that is a spherical clump with a diameter of about 0.1 mm. Either of the inside clump of cell grows to endoblast, mesoblast and ectoderm, is a portion forming all cells of the body, and the nutritional ectoderm forms their placentas and forms bags for separating the embryo from external filed. After unraveling the inside clump of cell, cell is taken out and the ES cell can be produced by culturing these cells at the environment capable of growth and without differentiation. Consequently, the ES cell can be produced from the embryo as described above.

The ES cell for a mouse is established by Evans et. al. (Evans et. al., 1981, Nature 292: 154-6) in 1981 and Martin et. al. (Martin GR et. al., 1981, Proc. Natl. Acad. Sci. 78: 7634-8) and is commercially available from Dainippon & Sumitomo Pharmaceutical Co., Ltd. as a commodity.

Further, the human ES cell is established by Thomson et. al. (Thomson et. al., Science, 1998, 282: 1145-7) in 1998 and available from WiCell Research facilities (WiCell Research Institute, web site: http://www. wicell. org/, Madison, Wisconsin State, USA).

In Japan, the research of the human ES cell is assigned as National Bio Resource Project, and the human ES cell established in Stem Cell Medical Research Center belonging to Regenerative Medical Science Research Center of Kyoto University is available in accordance with "Distribution Prescription of Human ES Cell in Regenerative Medical Science Research Institute" (enforced on November 26, 2003) under a predetermined condition such as the acquisition of recognition (approval) of Minister of Education and Science. At present, the human ES cell strains "KhES-1" (established in August, 2003), "KhES-2" (established in October, 2003) and "KhES-3" (established in October, 2003) are available.

The human ES cell strains KhES-1, KhES-2 and KhES-3 are established using extra embryo (fertile egg scheduled to be disposed without transplantation) that was prepared for auxiliary reproductive medicine such as external fertilization and microscopic fertilization. Namely, they are established by a method by which the fertile egg is cultured to the stage of blastocysts, the inside clump of cell is separated from the blastocysts and cultured on feeder cells, the undifferentiated cells proliferated is selected and passaged repeatedly, to be able to be passaged. The establishment method itself is not essentially changed from the cases of a mouse and a monkey. The human ES cells prepares a flat and nearly single layer colony on the feeder cells that is different from the mouse ES cells, and is very similar morphologically to the monkey ES cells. The human ES cells expresses a marker peculiar to the undifferentiated ES cells and keeps a normal chromosome composition (refer to Hirohumi Suemori, Clinical Pathology 52:3 (2004), pp254 to 258). As the marker peculiar to the undifferentiated ES cells expressed by the human ES cells, alkaline phosphatase (ALP), SSEA-3 and 4 and TRA-1-60 and 81 are recognized and the expression of NanogOct-3/4 and Rex-1 is confirmed by RT-PCR. Concerning karyotype (according to a G-band method), KhES-1 and KhES-2 are normal diploid and have the female karyotype and KhES-3 is normal diploid and has the male karyotype (refer to "Concerning Distribution of Human ES Cell -Human ES Cells KhES-1, 2 and 3--" in the home page of Stem Cell Medical Research Center belonging to Regenerative Medical Science Research Center of Kyoto University).

In the present invention, passage, retention and proliferation are carried out under a condition not differentiating the ES cells if necessary (hereinafter, the step is occasionally called as the preparation step) and differentiating induction to the hepatocytes is carried out by using its portion.

In the preparation step, it is preferable to use a container coated with feeder cells or scaffold from the viewpoint of growth ability, as the container carrying out the culture of the ES cells. Example of the feeder cells is the mouse embryonic fibroblasts treated with γ-ray irradiation. Further, the scaffold includes laminin, fibronectin, collagen (such as collagen IV, collagen I), entactin, peptide hydrogel having self-assembly ability, poly(p-N-vinylbenzyl-D-lactonamide) (PVLA) (polystyrene derivative having lactose at side chains). Commercially available products include Matrigel (comprising 56 % of laminin, 30 % of collagen IV and 8 % of entactin; manufactured by Becton Dickinson & Company), Growth Factor Reduced Matrigel (GFR Matrigel comprising 61 % of laminin, 39 % of collagen IV and 7 % of entactin; manufactured by Becton Dickinson & Company), Pura Matrix (Pura Matrix; peptide hydrogel being oligopeptide having 16 residual groups of amino acid and a length of about 5 nm; manufactured by Three Day Matrix Japan Co.), Poly(p-N-vinylbenzyl-D-lactonamide) (PVLA) (manufactured by Celagix Res Ltd.), etc. In case of the ES cells derived from a mouse, it is preferable to use a culture container coated with feeder cells. Further, in case of the ES cells derived from human, when it is used for the bioartificial liver being the one embodiment of the present invention, it is preferable to use a culture container coated with the scaffold without using the culture container coated with feeder cells derived from a mouse because of the fear of contamination of cells derived from foreign organism. Further, since the culture state of the human ES cells is good, the fore-mentioned Matrigel, Growth Factor Reduced Matrigel (GFR Matrigel), Pura Matrix are more preferably used.

Usual methods can be adopted as a culture solution and culture condition at the preparation step. The example of the culture solution includes (1) a culture solution in which 15 % fatal bovine serum (FBS), 1 % non essential amino acid, 1 % nucleotide, 110 µM of 2-mercaptoethanol (available from Dainippon & Sumitomo Pharmaceutical Co., Ltd.), 1 % penicillin and streptomycin, 1 % glutamic acid and 500 U/ml mouse derived leukemia inhibiting factor (LIF) (available from Dainippon & Sumitomo Pharmaceutical Co., Ltd.) are supplemented to a mix solution (a volume ratio of 1 : 1) of the ES culture solution (R-ES-101, bought from Dainippon & Sumitomo Pharmaceutical Co., Ltd.) and Dulbecco's Modified Eagle (DMEM) culture solution, (2) a medium for the primate ES cell (manufactured by Reprocell Inc.), (3) a naturalization medium for the mouse embryonic fibroblast (manufactured by R & D System Co.), etc. The culture temperature is preferably a range of 36 to 37°C and pH is preferably a range of 7.3 to 7.4. At the preparation step of the mouse ES cell, the leukemia inhibiting factor (LIF) is not effective for inhibiting differentiation and scaffolds such as Matrigel are preferably used. Further, as the culture method of not using the feeder cells but using the scaffold, a method (Protocols for the Maintenance of Human Embryo-stem cells in Feeder Free Conditions) disclosed by GERON Corporation. (Melono Park, California State, USA) is preferable.

In the method of the present invention, the concentration of dHGF used in the step of differentially inducing the ES cell to the hepatocyte is preferably higher than 1 ng/ml and lower than 1000 ng/ml, more preferably 10 ng/ml to 500 ng/ml and most preferably 100 ng/ml. When the concentration of dHGF is at most the fore-mentioned range, differentiation to the hepatocyte tends to be not achieved and on the other hand, when it exceeds the range, slight cellular disorder tends to be confirmed.

In the present invention, the differentiating induction may be carried out by directly adding dHGF for ES cells but after embryoid body (EB) is formed from the ES cells, dHGF is preferably added to carry out differentiating induction. The method of forming the Embryoid Body includes a hanging drop method by which the ES cells diluted about 105 cells/ml by a culture solution are sowed at the inside of the lid of a petri dish in liquid droplet shape (about 30 µl per one droplet) and cultured for about 2 days in a state in which the liquid droplets do not drop while hanging, the embryoid body is formed and then, the embryoid body is washed down with the culture solution and suspension-cultured in the petri dish to be ripened; a formation method by suspension culture by which the ES cells of about 5 × 10⁶ cells/ml are suspension-cultured in about 10 ml of the culture solution in the 10 cm petri dish for about 5 days to form the embryoid body. The method of forming the embryoid body by the suspension culture is more preferable.

In the present invention, it is preferable that after the culture is generally carried out for about 3 to 7 days at the formation step of the embryoid body, operation is transferred to the next step of differentiating induction.

As the culture solution and culture condition at the step of forming the embryoid body, a usual method can be adopted, and for example, the culture solution includes (1) an ES differentiating induction medium (DMEM F12) [a medium in which DMEM (GIBCO BRL, manufactured by Invitrogen Corporation) and a nutrition mixture F-12 (ham) (GIBCO BRL, manufactured by Invitrogen Corporation) are mixed at a proportion of volume ratio of 1 : 1, 4.5 mg/ml of glucose, 20 % FBS, 2 mM of L-glutamine, 25 mM of HEPES (GIBCO BRL, manufactured by Invitrogen Corporation) and 100 mg/ml of penicillin, 100 mg/ml of streptomycin (manufactured by Sigma-Aldrich Corporation) are added and LIF is not added], (2) a medium for primate ES cell (manufactured by Reprocell Inc.), (3) a naturalization medium for the mouse embryonic fibroblast (manufactured by R & D System Co.). The culture temperature is preferably a range of 36 to 37°C and pH is preferably a range of 7.3 to 7.4.

In the present invention, either of plate culture and three-dimensional culture can be used for the culture step of carrying out the differentiating induction, but it has been recently known that condition nearer to environment in organism can be prepared by carrying out the three-dimensional culture of cell on a scaffold (staging ground) and cell function can be improved; therefore the three-dimensional culture is preferable from the viewpoint of the improvement of liver function.

The scaffold used for the three-dimensional culture includes laminin, fibronectin, collagen such as collagen IV and collagen I, entactin, peptide hydrogel, poly(p-N-vinylbenzyl-D-lactonamide) (PVLA). Commercially available products include Matrigel, Growth Factor Reduced Matrigel (GFR Matrigel), Pura Matrix. Further, the non woven cloth described below can be used.

As the culture solution and culture condition at the step of the differentiating induction, a usual method can be adopted, and for example, the culture solution includes (1) an ES differentiating induction medium (DMEM F12) [a medium in which DMEM (GIBCO BRL, manufactured by Invitrogen Corporation) and a nutrition mixture F-12 (ham) (GIBCO BRL, manufactured by Invitrogen Corporation) are mixed at a proportion of volume ratio of 1 : 1,4.5 mg/ml of glucose, 20 % FBS, 2 mM of L-glutamine, 25 mM of HEPES (GIBCO BRL, manufactured by Invitrogen Corporation) and 100 mg/ml of penicillin, 100 mg/ml of streptomycin (manufactured by Sigma-Aldrich Corporation) are added and LIF is not added], (2) a medium for primate ES cell (manufactured by Reprocell Inc.), (3) a naturalization medium for the mouse embryonic fibroblast (manufactured by R & D System Co.). The culture temperature is preferably a range of 36 to 37°C and pH is preferably a range of 7.3 to 7.4. The culture period is about 14 to 21 days.

In the differentiating induction step of the present invention, at least one or two of various organic solvents, cytokine, hormone can be used in combination in addition to dHGF. Any one of the organic solvents, cytokine, hormone can be used so far as they do not provide bad influence, but those have been conventionally used for the differentiation of cells are preferable. The specific example includes organic solvents such as dimethylsulfoxide (DMSO), dimethylacetoamide (DMA), hexamethylene bis(acetoamide) and other polyethylene bis(acetamide); activin A, bFGF, glucocorticoid, epithelial growth factor (EGF), insulin, TGF-α, TGF-β, FGF, heparin, dexamethasone; and cytokine and hormones such as HGF, IL-1, IL-6, IGF-I, IGF-II and HBGF-1.

The hepatocytes differentially induced from ES cells by the method of the present invention can be confirmed by the observation of morphological characteristics and reverse transcription polymerase chain reaction (RT-PCR) .

The morphological confirmation methods include the confirmation of morphological characteristics specific for the hepatocyte such as cells having a plurality of nuclei observed by a phase microscope and granules rich in cytoplasm observed by an electron microscope, in particular, the presence of glycogen granules.

The trait expression of the hepatocyte induced can be evaluated by the RT-PCR. If the expression of albumin gene and the no expression of α-fetoprotein being undifferentiated gene can be confirmed by RT-PCR , it can be said that the differentiation is induced. Further, it is preferable that as an index representing the degree of maturation as the hepatocyte, the genetic expression of CK 18 (cytokeratin 18) being the marker of epithelial cell, G6P (glucose hexaphosphorylated enzyme), TAT (tyrosine aminotransferase) and HNF3b (nuclear factor 3β of the hepatocyte) is recognized.

Further, whether it can be really functioned as the hepatocyte or not is a great point whether metabolism can be carried out by adding ammonia, lidocaine, diazepam to a medium or not. Further, if albumin and urea are produced in the medium, it can be said that it is differentiated to adequately functionable hepatocyte.

In the present invention, after the ES cell is grown to the necessary number of cells, the differentiating induction to the hepatocyte is carried out using dHGF; therefore a large quantity of the safe hepatocyte can be fed without requiring the introduction of a gene. The development of the bioartificial liver from which all people receive benefit can be greatly expected by using this as the cell source of the bioartificial liver.

Accordingly, another embodiment of the present invention is the bioartificial liver containing the hepatocyte differentially induced from the ES cell and a production process thereof.

When the bioartificial liver of the present invention is produced, it is considered that the degree of the differentiation of cells at charging in a reactor is important. The ES cell reinforces adherence to scaffold such as a non-woven cloth by forming the embryoid body. Further, cells adhering on the scaffold such as a non-woven cloth exhibit the formation of three-dimensional spheroid. It has been recently known that condition near to environment in organism can be made by three-dimensionally culturing cells on scaffold and cell function is improved; therefore it is effective for the improvement of function that cells are cultured on the scaffold such as a non-woven cloth. Consequently, the metabolic ability of ammonia that is specific for the hepatocyte and is high function is greatly reinforced by filling the ES cell in a reactor after forming the embryoid body and carrying out the differentiating induction at three-dimension and by plate culture.

Consequently, it is preferable that after the formation of the embryoid body from the ES cell is promoted, cells are filled in a reactor and the differentiation to the hepatocyte by the induction method of the present invention is carried out.

The bioartificial liver includes a hybrid type bioartificial liver combining a hollow fiber type reactor (device) with separation and culture cell. The bioartificial liver has three morphologies of the bioartificial liver externally installed and connected to the blood vessel, that indwelled in the body and connected with the blood, and that indwelled in the abdominal cavity without being connected with the blood vessel. The hepatocyte of the present invention can be used for any morphologies of the bioartificial livers, but the external type is preferable from the viewpoint of preventing dangerousness accompanied with cell transfusion because when the ES cell is differentially induced, growth ability is lost but when the ES cell whose differentiation was inadequate remains, it is more likely to develop teratoma when the cell is transplanted in the living organism.

The design and development of the reactor are also important elements in the development of the bioartificial liver. As the bioreactor, there are known various types such as hepato assist for the bioartificial liver treatment (Hui T. Rozga, J. Demetriou AA., J. Hepatobiliary Pancreat Surg. 2001; 8:1-15) using porcine hepatocyte that is centrally carried out by Demetriou of Cedars-Sainai Medical Center (Los Angeles, California State, USA) under the support of Circe Biomedical Inc. (Lexington, Massachusetts State, USA); MELS (Modular Extracorporeal Liver System) by Gerlach in Germany using porcine hepatocyte. These reactors can be also used in the present invention, but since there is no scaffold to which the hepatocyte adheres, the cell is only filled in space in the hollow fiber or space out of the hollow fiber and tends to be in a floated state. The hepatocyte tends not to adequately express the differentiation function and collides with peripheral cells to be easily subject to stressful stimuli.

Accordingly, in the present invention, a reactor comprising scaffolds such as the hollow fiber and non-woven cloth are preferable so as to provide staging ground for cell.

Any one of the hollow fiber membranes can be used so far as it does not occur that cell adheres on membrane surface and substance exchange is not prevented. Specifically, commercially available membranes that have been conventionally used for medical uses, for example, polysulfone membrane, the saponified product membrane of an ethylene-vinyl acetate random copolymer (for example, trade name: EVAL, manufactured by Kuraray Medical Co., Ltd.) are preferable. The pore size of the commercially available hollow fiber membrane includes dialysis membrane (about up to 5 nm), the separation membrane of plasma component (20 to 30 nm), the separation membrane of plasma (30 to 200 nm). The separation membrane of plasma is preferable from the viewpoint of the permeability of a substance. In order to prevent the dangerousness of rejection symptom, 30 nm to 100 nm is most preferable so as not to be directly brought in contact with cells filled on the scaffold such as non-woven cloth out of the hollow fiber.

As the non-woven cloth, those processed and modified so that cells can adhere are preferable. The fiber of the non-woven cloth includes poly(tetrafluoroethylene) (PTFE). In particular, those in which polyamino acid urethane (PAU) processing was carried out for the poly (tetrafluoroethylene) (PTFE) are preferable from the viewpoint of easy processing.

A process till housing is shown in FIG.3 exemplifying the embodiment of the bioartificial liver of the present invention. A hollow fiber 7 is placed on a non-woven cloth 6 to which backing 5 was performed (FIG.3 (a)). Hereat, the non-woven cloth 6 to which backing 5 was performed is provided with a slit 8. This is wound up in a roll shape (FIG.3 (b)). Its X-X line section is FIG.3 (c). This is assembled in a tubular container 10 on both ends of which liquid leak-preventing members 9 are provided. The reactor 11 is provided with a cell injecting orifice 12 and a taking-out orifice 13 from which the sampling of cell sample can be carried out, and the slit 8 is arranged so as to be linked with the cell injecting orifice 12.

Further, the bioartificial liver therapy is preferably carried out by a device integrating functions that enable 1) the monitoring of the flow-in pressure and flow-out pressure of the bioartificial liver at real time, 2) the actuation of alarm at generation of foams, 3) the warming up of the reactor (at 37°C) in order to be safely and scientifically carried out.

Porcine hepatocyte newly separated and reversible immortalized human hepatocyte are filled in a hollow fiber and non-woven cloth type reactor as the bioartificial liver and good result is obtained in animal experiment in monkey hepatic disorder model (Naoya Kobayashi et. al., "Hybrid Artificial Liver" 2003, Liver Gallbladder Pancrea Vol.46, pp381-393).

Further, the hepatocyte, in particular, the human hepatocyte differentially induced from the ES cell by the method of the present invention is useful as cell for the metabolic test of a drug. Namely, it is useful as cell for test such as in the development of a new drug, safety test, toxicity test.

The hepatocyte is the center of various drug metabolisms and it is necessary to study the metabolism by administrating a drug to the hepatocyte in order to test the toxicity and safety of the drug. Further, since there is great difference between a human and a test animal in substance metabolism, test using the hepatocyte is indispensable for the final test. The present invention capable of providing a large quantity of the human hepatocyte with uniform quality is useful. Those having a structure similar as the bioartificial liver can be used as a device used for such metabolism test.

Further, the hepatocyte, in particular, the human hepatocyte differentially induced from the ES cell by the method of the present invention can be used for production of a biologically active substance. The biologically active substance includes albumin, various blood coagulation factors. Those having a structure similar to the bioartificial liver can be used as the production device of the biologically active substance.

The present invention is illustrated below according to Examples using the ES cell derived from a mouse and a human but the present invention is not limited to these.

### EXAMPLES

### PRODUCTION EXAMPLE 1

### Preparation of ES cell

The ES cells derived from 129v mice (bought from Dainippon & Sumitomo Pharmaceutical Co., Ltd.) were cultured using feeder cells (the mouse-derived embryonic fibroblast; bought from Dainippon & Sumitomo Pharmaceutical Co., Ltd.) in which neomycin resistance gene was introduced, using a culture flask T-75 (manufactured by Falcon Co. and sold by Becton Dickinson & Company) on which aqueous gelatin with a concentration of 0.1 % (Catalogue No.: R-ES-006B, bought from Dainippon & Sumitomo Pharmaceutical Co., Ltd.). As the culture solution, a culture solution in which 15 % fatal bovine serum (FBS), 1 % non essential amino acid, 1 % nucleotide, 110 µM of 2-mercaptoethanol (available from Dainippon & Sumitomo Pharmaceutical Co., Ltd.), 1 % penicillin and streptomycin, 1 % glutamic acid and 500 U/ml mouse derived leukemia inhibiting factor (LIF) (available from Dainippon & Sumitomo Pharmaceutical Co., Ltd.) were supplemented to a mix solution (a volume ratio of 1 : 1) of the ES culture solution (R-ES-101, bought from Dainippon & Sumitomo Pharmaceutical Co., Ltd.) and Dulbecco's Modified Eagle (DMEM) culture solution was used. The culture solution was replaced every day and the ES cells were passaged by every three days. When a proportion at which the ES cell occupies in a culture plate was a state of 80 to 90 %, the ES cells remaining in the culture plate were collected by 2 stages treatment, namely, by firstly adding trypsin-EDTA (GIBCO BRL, manufactured by Invitrogen Corporation) of a concentration of 0.25 %, removing the feeder cell together by removing the trypsin-EDTA solution after the lapse of time for 45 sec (the ES cells remain yet in the culture plate at this stage) and then, adding the ES culture solution and peeling it after 2 min.

### EXAMPLE 1

The differentiating induction culture of 5 million cells of the ES cells obtained in Production Example 1 to the hepatocyte was carried out by adding mouse derived dHGF of a concentration of 100 ng/ml (presented by Dai-ichi Kogyo Seiyaku Co., Ltd. (Tokyo)), bFGF of a concentration of 100 ng/ml, DMSO of a concentration of 1 % (manufactured by Sigma-Aldrich Corporation) and dexamethasone of a concentration of 10⁻⁷ mol (manufactured by Sigma-Aldrich Corporation) in 10 ml of an ES differentiating induction medium (DMEM F12) in a culture flask T-75 (manufactured by Falcon Co. and sold by Becton Dickinson & Company) coated with aqueous gelatin of a concentration of 0.1 % (Catalogue No.: R-ES-006B, bought from Dainippon & Sumitomo Pharmaceutical Co., Ltd.).

The composition of the DMEM F12 was a medium in which DMEM (GIBCO BRL, manufactured by Invitrogen Corporation) and a nutrition mixture F-12 (ham) (GIBCO BRL, manufactured by Invitrogen Corporation) was at a proportion of 1 : 1, 4.5 mg/ml of glucose, 20 % FBS, 2 mM of L-glutamine, 25 mM of HEPES (GIBCO BRL, manufactured by Invitrogen Corporation), 100 mg/ml of penicillin, 100 mg/ml of streptomycin (manufactured by Sigma-Aldrich Corporation) were added and LIF was not added. The culture solution was replaced by every three days. When the study of the differentiating induction effect to the hepatocyte was carried out after 14 days from the start of culture, the cell had the so-called morphological characteristic of the hepatocyte called as nuclei having several nucleous and the expression of albumin was observed; therefore differentiation to the hepatocyte was confirmed.

### EXAMPLE 2

The dispersion culture of 5 million cells of the ES cells obtained in Production Example 1 was carried out with a 10 cm sterilized petri dish (shallow type of 90 × 15 mm and commodity number; SH90-15, manufactured by Asahi Technoglass Corporation) for 5 days using 10 ml of the ES culture solution (R-ES-101), to form embryoid body.

Then, the mouse hepatocytes were induced in like manner as Example 1 except that the embryoid body was used in place of the ES cells.

### EXAMPLE 3

The embryoid body formed in like manner as Example 2 was sowed in a culture plate with 12 wells (formal name: Multiwell 12-well Falcon; manufactured by Falcon Corporation and sold by Becton Dickinson & Company) on which a polytetrafluoroethylene non-woven cloth (manufactured by Kuraray Medical Inc.) processed with poly(amino acid urethane) (PAU) that has cell adhering property with a size of 1 cm × 1 cm, so as to be 3 × 10⁶ cells/cm², and the ES differentiating induction medium (DMEM F12) same as Example 1 was used, the mouse derived dHGF of a concentration of 100 ng/ml (presented by Dai-ichi Kogyo Seiyaku Co., Ltd. (Tokyo)), bFGF of a concentration of 100 ng/ml, DMSO of a concentration of 1 % (manufactured by Sigma-Aldrich Corporation) and dexamethasone of a concentration of 10⁻⁷ mol (manufactured by Sigma-Aldrich Corporation) were added and it was cultured for 14 days to induce the mouse hepatocyte. The culture solution was replaced by every three days.

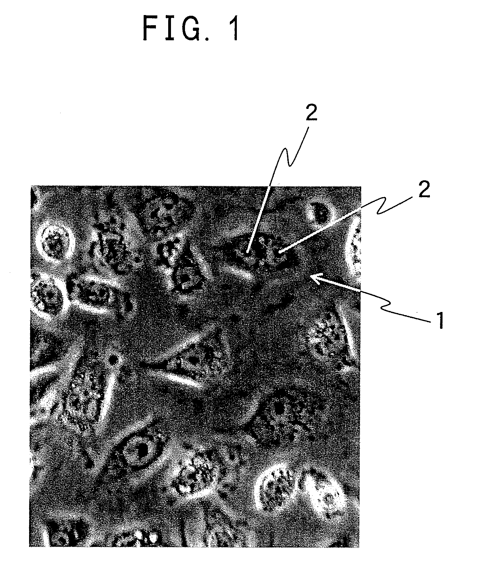

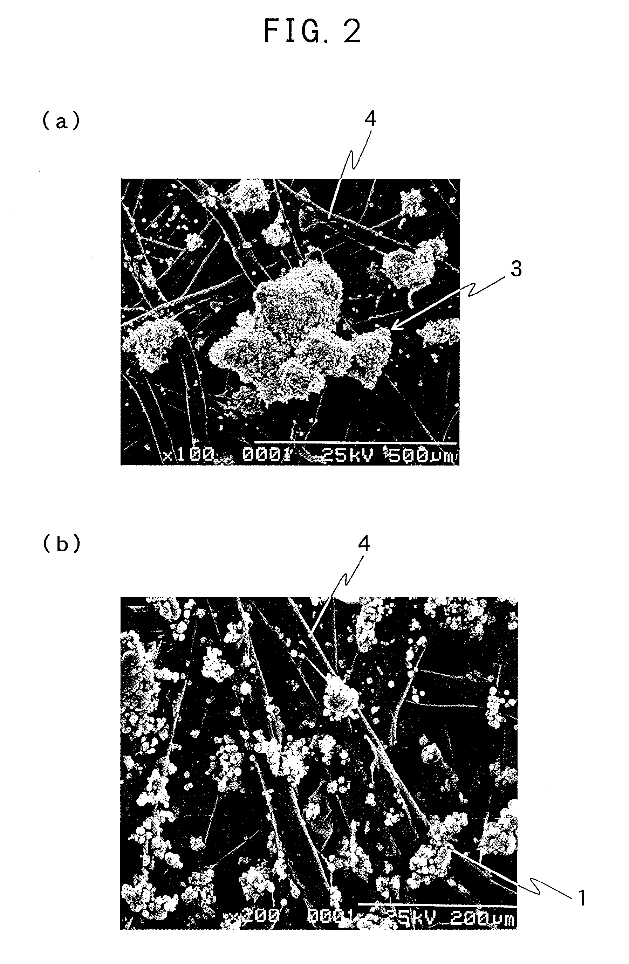

The conformation of the cells obtained was observed using a phase microscope (Olympus CK40, manufactured by Olympus Imaging Corporation) (FIG.1). Further, the adhering situation (FIG.2) of the embryoid body just after sowing to a non-woven fiber 4 and the hepatocyte (FIG.2(b)) differentially induced after 14 days were also observed. The code 1 shows the hepatocyte differentially induced, the code 2 shows nuclei, the code 3 shows the embryoid body and the code 4 shows the non-woven fiber.

### PRODUCTION EXAMPLE 2

### Production of bioartificial liver reactor

The bioartificial livers (BAL) (refer to FIG.3) of a whole blood perfusion type system comprising a hollow fiber and a non-woven cloth were produced in accordance with the procedure below. They were respectively referred to as BAL-1 to BAL-3 depending on the kind of the hollow fiber membranes as shown in Table 1.

**TABLE 1**

| bioartificial liver | hollow fiber membrane | |
|---|---|---|
| | material | pore size |
| BAL-1 | polysulfone *1 | dialysis membrane |
| BAL-2 | polysulfone | plasma separation membrane |
| BAL-3 | polysulfone | plasmapheresis membrane |

| | | |
|---|---|---|
| * Manufactured by Kuraray Medical Co., Ltd. | | |

550 of hollow fiber membranes with a length of 10 cm were regularly placed on a non-woven fiber (10 × 10 cm) backed with rayon (refer to FIG.3(a)) and this was wound up in a roll shape (refer to FIG.3(b)). The schematic view of the section is shown in FIG.3(c). The roll comprising the non-woven cloth and the hollow fiber membranes was assembled in a tubular container on both ends of which liquid leak-preventing members were provided (refer to FIG.3(d)).

Further, as the non-woven cloth, a PTFE non-woven cloth (manufactured by Kuraray Medical Inc.) processed with poly (amino acid urethane) (PAU) that has cell adhering property was used.

When respective reactors were installed between the artery and the vein in the neck of healthy pig and actuated in order to judge the biocompatibility of the reactors obtained, they could be operated for 24 hours without badly affecting the circulatory dynamics without accident. Further, the reduction of hemocyte components such as the blood erythrocyte and blood platelet was not confirmed.

Further, in order to safely and scientifically carry out BAL treatment, a device integrating functions that enable 1) the monitoring of the flow-in pressure and flow-out pressure of the BAL reactor at real time, 2) the actuation of alarm at generation of foams, 3) the warming up of the reactor (at 37°C) has been developed and it was verified by the pig experiment that the system is actuated well.

### EXAMPLE 4

The embryoid body formed Example 2 was filled in space out of a hollow fiber in the bioartificial liver reactor BAL-2 obtained in Production Example 2, and the mouse derived dHGF of a concentration of 100 ng/ml (presented by Dai-ichi Kogyo Seiyaku Co., Ltd. (Tokyo)), the mouse derived bFGF of a concentration of 100 ng/ml, DMSO of a concentration of 1 % (manufactured by Sigma-Aldrich Corporation) and dexamethasone of a concentration of 10⁻⁷ mol (manufactured by Sigma-Aldrich Corporation) were added in 10 ml of the ES differentiating induction medium (DMEM F12), to carry out the differentiating induction culture to the hepatocyte. The culture solution was replaced by every three days and the effect of the differentiating induction was studied after 14 days from the culture.

### TEST EXAMPLE 1

### Gene expression in hepatocyte induced

The expression of important genes related to hepatic metabolism, namely CK18 (cytokeratin 18), G6P (glucose hexaphosphorylated enzyme), TAT (tyrosine amino acid transferase), albumin, AFP (α-fetoprotein) and HNF3b (nuclear factor 3β of hepatocyte) genes were tested by the RT-PCR method in the hepatocytes induced (Examples 2 and 3). The expression of GAPDH gene was tested as an endogenous control. In the RT-PCR method, after cells were treated with 0.25 % trypsin-EDTA (GIBCO BRL, manufactured by Invitrogen Corporation), they were collected and RNA was extracted according to the instruction manual of the product using RNA TRIZOL (manufactured by Invitrogen Corporation). The reverse transcription reaction of 1 µg of total RNA was carried out at 22°C for 10 min and at 42°C for 20 min using RNA reverse transcription gene.

2 µg of reverse transcription product obtained was used for PCR growth according to its protocol using an AmpliTag Gold kit (Perkin Elmer/Cetus Co.; NoWork, Connecticut State, USA) at 20 pmol/ml of each of primers. The primers for respective genes and PCR condition are described below.
Mouse CK18 gene (182bp, 59°C and 35 cycles)
5'Primer: 5'-CGATACAAGGCACAGATGGA (SEQ ID No. 1)
3'Primer: 5'-CTTCTCCATCCTCCAGCAAG (SEQ ID No. 2)
Mouse G6P gene (206bp, 57°C and 35 cycles)
5'Primer: 5'-CAGGACTGGTTCATCCTT (SEQ ID No. 3)
3'Primer: 5'-GTTGCTGTAGTAGTCGGT (SEQ ID No. 4)
Mouse tat gene (216bp, 55°C and 35 cycles)
5'Primer: 5'-ACCTTCAATCCCATCCGA (SEQ ID No. 5)
3'Primer: 5'-TCCCGACTGGATAGGTAG (SEQ ID No. 6)
Mouse albumin gene (174bp, 59°C and 35 cycles)
5'Primer: 5'-GCTAGGCACACAGTGCTTG (SEQ ID No. 7)
3'Primer: 5'-CAGGATTGCAGACAGATAGTC(SEQ ID No. 8)
Mouse AFP gene (300bp, 59°C and 32 cycles)
5'Primer: 5'-CACTGCTGCAACTCTTCGTA (SEQ ID No. 9)
3'Primer: 5'-CTTTGGACCCTCTTCTGTGA (SEQ ID No. 10)
Mouse HNF3b gene (500bp, 60°C and 35 cycles)
5'Primer: 5'-TATTGGCTGCAGCTAAGCGG (SEQ ID No. 11)
3'Primer: 5'-GCATCGGACTCAGGTGAGGT (SEQ ID No. 12)
Mouse GAPDH gene (459bp, 60°C and 32 cycles)
5'Primer: 5'-CAGCCGAGCCACATC (SEQ ID No. 13)
3'Primer: 5'-TGAGGCTGTTGTCATACTTCT (SEQ ID No. 14)

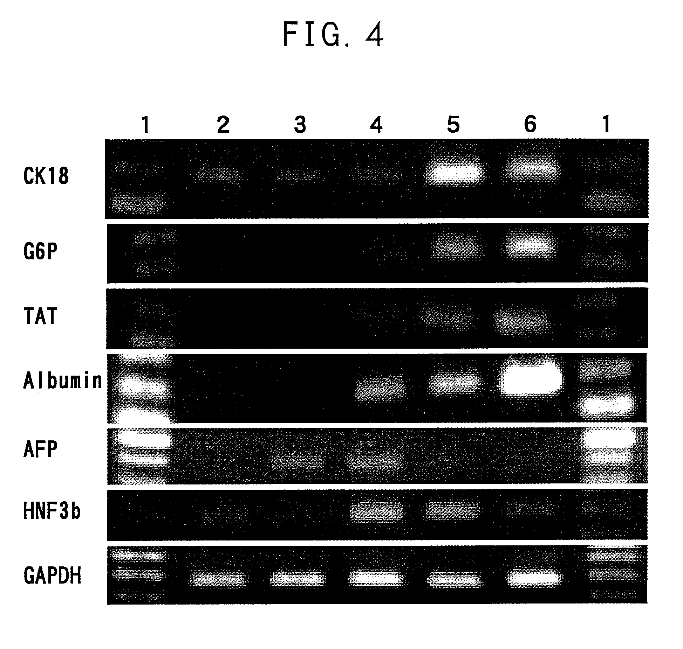

The result is shown in FIG.4. Lanes 1 to 6 show respectively the size marker, undifferentiated ES cell, feeder cell, differentially induced ES cell (Example 2), ES cell differentially induced on a non-woven cloth (Example 3) and mouse hepatocyte. In the hepatocyte induced in Examples 2 and 3, the expression of important genes related to hepatic metabolism, CK18 (cytokeratin 18), G6P (glucose hexaphosphorylated enzyme), TAT (tyrosine amino acid transferase), albumin, and HNF3b (nuclear factor 3β of hepatocyte) genes is confirmed, but in particular, in the hepatocyte differentially induced using a non-woven cloth (Example 3), the expression of CK18 (cytokeratin 18), G6P (glucose hexaphosphorylated enzyme), TAT (tyrosine amino acid transferase) and albumin genes is enhanced, and the disappearance of the expression of AFP (α-fetoprotein) that is the marker of undifferentiated cell was observed. The result shows that the differentiating induction to the hepatocyte is more efficiently induced by using a non-woven cloth.

### TEST EXAMPLE 2

### Measurement of hepatic function

### (A) Metabolism of ammonia

The culture solutions of the hepatocytes obtained in Examples 2 to 4 were replaced with new culture solution after 14 days from the start of the differentiating induction. In the culture solutions, 1.4 mmol of ammonium tetrachloride was loaded, 10 µl of the culture solution was sampled after 4 hrs and the concentration of ammonia was measured.

### (B) Metabolism of diazepam

The culture solutions of the hepatocytes obtained in Examples 2 to 4 were replaced with new culture solution after 14 days from the start of the differentiating induction. In the culture solutions, diazepam was loaded so as to be a final concentration of 1 µg/ml, 1000 µl of the medium was sampled after 4 hrs and the concentration of diazepam was measured.

### (C) Metabolism of lidocaine

The culture solutions of the hepatocytes obtained in Examples 2 to 4 were replaced with new culture solution after 14 days from the start of the differentiating induction. In the culture solutions, lidocaine was loaded so as to be a final concentration of 1 mg/ml, 1000 µl of the medium was sampled after 4 hrs and the concentration of lidocaine was measured.

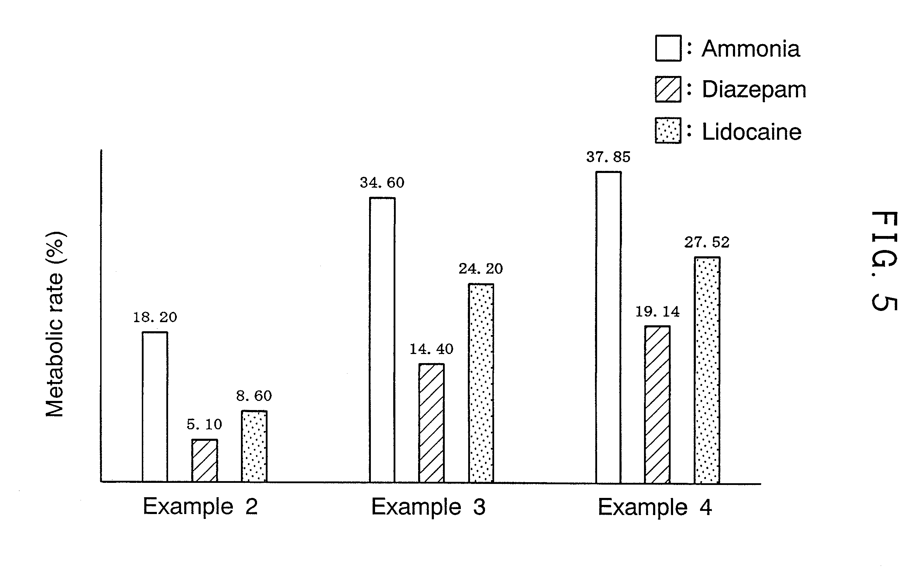

The metabolic rate of each of the fore-mentioned (A) to (C) is shown in FIG.5. The metabolic rate is represented by dividing difference obtained by subtracting the concentration of each of drugs after 4 hrs from the concentration of a drug at loading the drug, by the concentration of the drug at loading the drug. Drug metabolic ability is observed in each of Examples, but it is grasped that the drug metabolic ability is enhanced by culture in a non-woven cloth and further by three-dimensional culture in the reactor.

### (D) Production of urea

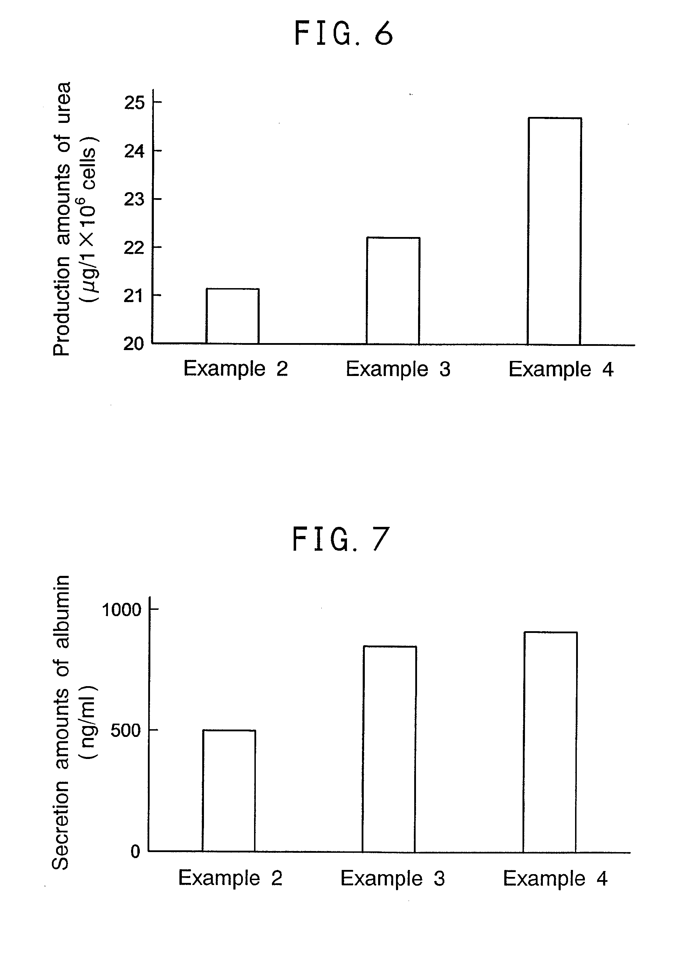

1.4 mmol of ammonium tetrachloride was loaded in like manner as the fore-mentioned (A), the culture solution was sampled after 4 hrs and the production amount of urea was measured. The production amounts were respectively 21.4 µg, 22.2 µg and 24.7 µg for Examples 2 to 4 (FIG.6).

### (E) Production of albumin

The culture solutions of the hepatocytes obtained in Examples 2 to 4 were replaced with new culture solution after 14 days from the start of the differentiating induction. Then, the culture solutions after culturing for 24 hours were sampled and the production of albumin was measured using a mouse albumin ELIS A kit (commodity code: E90-134, Funakoshi Corporation). The production amounts of albumin were respectively 500 ng, 850 ng and 920 ng for Examples 2 to 4 (FIG.7). It is grasped that the albumin production ability is enhanced by culture on a non-woven cloth and further by three-dimensional culture in the reactor.

### REFERENCE EXAMPLE

Functional evaluation of bioartificial liver filled with fresh porcine hepatocyte

The safety and effectiveness of the bioartificial liver reactor prepared in Production Example 1 using a crab-eating monkey (bought from Clea Japan) out of quarantine in accordance with the handling guide line of facility animal experiment.

About one billion porcine hepatocytes (existence rate > 90 %) were filled in the bioartificial liver reactor BAL-1 that was prepared in Production Example 1. The freshly separated porcine hepatocytes separated region out of the liver surgically cut by a four stage back flow method using disperse and collagenase (Masanobu Maruyama, Naoya Kobayashi and Toshinori Totsugawa et. al. Organ Biology, Vol.9, No.3, pp295-301, 2002).

To male crab-eating monkeys (4 kg, n = 6), 0.5 g/kg of D-galactosamine (bought from Sigma Co., Ltd.) was intravenously injected to induce drug-induced hepatic disorder.

After 18 hrs from the administration of D-galactosamine, atom 6Fr catheters (Atom Medical Co., Tokyo Japan) were inserted into the left internal carotid artery of the monkeys (n = 2) of a treatment group under general anesthetic, atom 6Fr catheters were inserted into the left external jugular vein and the bioartificial liver reactor filled with the porcine hepatocytes was connected between them. A PRISTA BIO-MINIPUMP (ATTO, Tokyo, Japan) was used as the pump, and the blood was externally circulated at a flow rate of 10 ml/min for 6 hours. Anesthesia was intravenous anesthesia and DIPRIVAN was used (5 ml/hr). As antithrombotic therapy, 1000 unit of heparin was injected from the central side of the reactor just before the blood passes the reactor and thereafter, the whole body was heparinized by the continuous injection of heparin during external circulation.

As negative control group (n = 2), similar test was carried out using a vacant reactor in which cells are not filled.

As a result, monkeys in 2 examples died because of hepatic disorder within 1 week after the administration of D-galactosamine. At autopsy, the bleeding necrosis and remarkable atrophia of the liver and the accumulation of ictero pleural effusion and ascites were confirmed and the broad necrosis of the hepatocyte was also confirmed histologically. On the other hand, either of the groups loaded with about one billion porcine hepatocytes was alive for at least 3 weeks and no abnormality was also confirmed by autopsy findings.

### PRODUCTION EXAMPLE 3

### Preparation of human ES cell

As the human derived ES cell, KhES-1 (distributed in accordance with "Distribution Prescription of Human ES Cell in Regenerative Medical Science Research Institute" from Regenerative Medical Science Research Center of Kyoto University) was used. The culture of the cells was carried out in accordance with the culture method disclosed by GERON Corporation (Protocols for the Maintenance of Human Embryo-stem cell in Feeder Free Condition, published in the web site (http://www.geron.com) of GERON Corporation). Specifically, the culture was carried out below.

As the culture plate, a culture plate with 6 wells (manufactured by Falcon Corporation and sold by Becton Dickinson & Company) coated with Matrigel (manufactured by Becton Dickinson & Company) was used and the culture solution used a naturalization medium for the mouse embryonic fibroblast (Catalogue No.: AR005; manufactured by R & D System Co.) as the ES culture solution. The culture solution was replaced every day and the mouse basic fibroblast growth factor (bFGF) was added to the culture solution so as to be 100 ng/ml at replacement. The ES cells were passaged by every seven days. Specifically, when a proportion at which the ES cells occupied in the culture plate was in a state of 80 to 90 %, feeder cells were removed by two stages treatment, namely that the collagenase IV solution (200 U/ml) (GIBCO BRL/ manufactured by Invitrogen Corporation; Catalogue No.: 17104-019) was firstly added and after the lapse of 10 min at 37°C, the collagenase IV solution was removed. At this stage, the ES cells in an undifferentiated state remained in the plate. Then, 2 ml of the ES culture solution was added to the 6 wells plate, the ES cells in an undifferentiated state remaining in the culture plate were scratched using a 2 ml pipette, then, pipetting was carried out 5 times and the cells were sowed on the 6 wells plate to be passaged.

### EXAMPLE 5

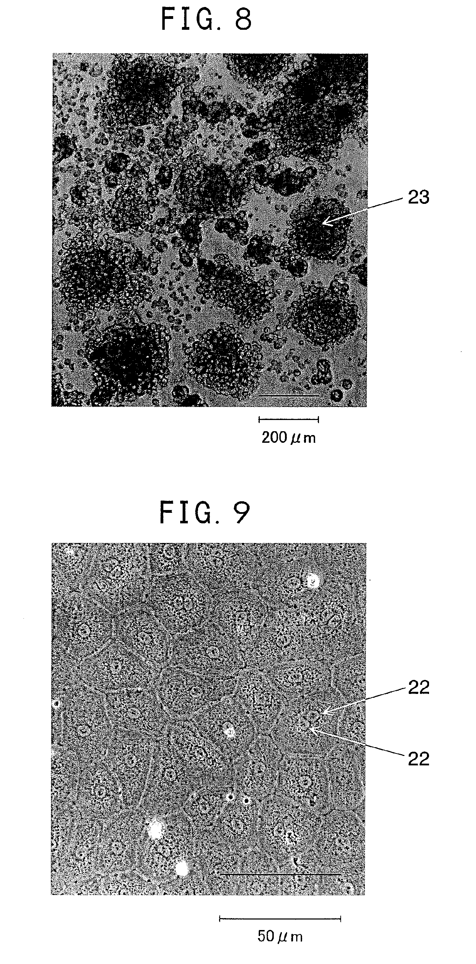

The solution in which 5 × 10⁶ KhES-1 cells obtained in Production Example 3 was diluted with 2 ml of the ES culture solution (same as the solution used in Production Example 3) was sowed on the center of a culture plate for forming embryoid body (COASTAR, manufactured by Corning Incorporated) in which 1 ml of the ES culture solution (same as the solution used in Production Example 3) was preliminarily added. Culture was carried out with the culture plate for forming embryoid body for 5 days to form the embryoid body and then, it was cultured with the ES culture solution (same as the solution used in Production Example 3) containing the human derived dHGF (presented by Dai-ichi Kogyo Seiyaku Co., Ltd.) of 100 ng/ml, the human derived bFGF of 100 ng/ml, DMSO of 1 % and dexamethasone of a concentration of 10⁻⁷ mol (manufactured by Sigma-Aldrich Corporation), to carry out the differentiating induction to the hepatocyte for 14 days. The culture solution was replaced by every two days. FIG.8 shows the phase microscope photograph of the humans ES cell forming the embryoid body and FIG.9 shows the phase microscope photograph of the hepatocyte differentially induced from the humans ES cell. A plurality of nuclei 22 being the characteristic of the hepatocyte were observed in the hepatocyte 21 differentially induced.

### EXAMPLE 6

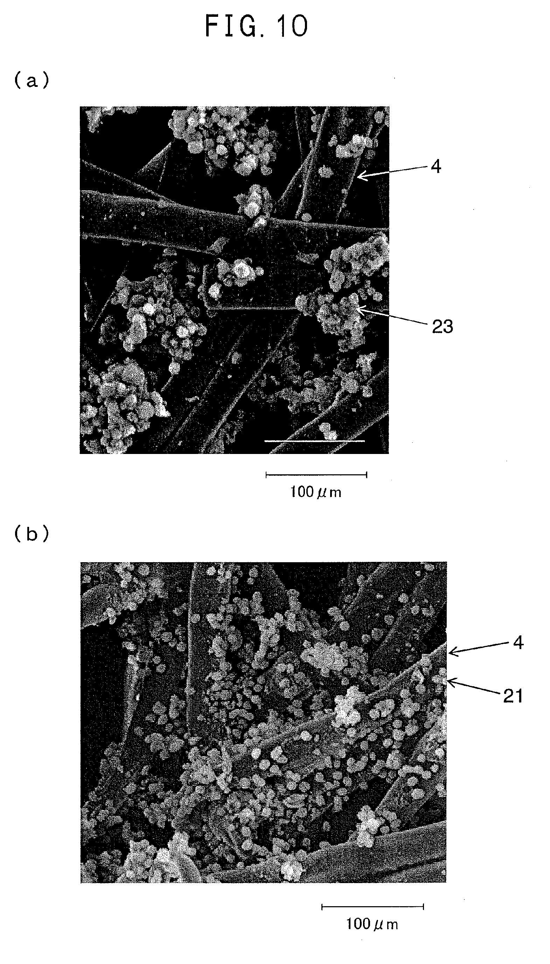

The KhES-1 cells obtained in Production Example 3 were sowed in a culture plate with 12 wells (Multiwell 12-well Falcon; manufactured by Falcon Corporation) on which a PTFE non-woven cloth (manufactured by Kuraray Medical Inc.) processed with poly(amino acid urethane) (PAU) that has cell adhering property with a size of 1 cm × 1 cm, so as to be 3 × 10⁶ cells/cm². After the ES cells were sowed on the culture plate, they were cultured for 5 days to form embryoid body. FIG.10(a) shows the scanning electron microscope photograph of the embryoid body formed on the non-woven cloth. FIG.10(a) shows the embryoid body 23 adhering on the fiber of the non-woven cloth 4.

Then, culture was carried out for 14 days using the ES culture solution (same as the solution used in Production Example 3) containing the human derived dHGF of 100 ng/ml, the human derived bFGF of 100 ng/ml, DMSO of 1 % and dexamethasone of 10⁻⁷ mol and the differentiating induction to the hepatocyte was carried out. The culture solution was replaced by every two days. FIG.10(b) shows the scanning electron microscope photograph of the hepatocyte differentially induced on the non-woven cloth. FIG.10(b) shows the hepatocyte 21 differentially induced that adhered on the fiber 4 of the non-woven cloth.

### TEST EXAMPLE 3

Gene expression in hepatocyte induced and morphological change

The expression of albumin gene being gene related to hepatic metabolism and specific to the hepatocyte was tested by the RT-PCR method in the hepatocytes described in Examples 5 and 6. The expression of β-actin gene was tested as an endogenous control. In the RT-PCR method, after cells were treated with 0.25 % trypsin-EDTA, they were collected and RNA was extracted according to the instruction manual of the product using RNA TRIZOL. The reverse transcription reaction of 1 µg of total RNA was carried out at 22°C for 10 min and at 42°C for 20 min using RNA reverse transcription gene.

2 µg of reverse transcription product obtained was used for PCR growth according to its protocol using an AmpliTag Gold kit (Perkin Elmer/Cetus Co.) at 20 pmol/ml of each of primers. The primers for respective genes and PCR condition are described below.
Human albumin gene (576bp, 59°C and 35 cycles)
5'Primer: 5'-AAACCTCTTGTGGAAGAGCC (SEQ ID No. 15)
3'Primer: 5'-CAAAGCAGGTCTCCTTATCG (SEQ ID No. 16)
Human β-actin gene (610bp, 60°C and 32 cycles)
5'Primer: 5'-TGACGGGGTCACCCACACTGTGCCCATCTA (SEQ ID No. 17)
3'Primer: 5'-CTAGAAGCATTTGCGGTGGACGATGGACGG (SEQ ID No. 18)

The result is shown in FIG.11. Lanes 1 to 7 show respectively the size marker, undifferentiated human ES cell, human ES cell forming embryoid body, differentially induced human ES cell without using on a non-woven cloth after forming the embryoid body (Example 5), human ES cell differentially induced on a non-woven cloth after forming the embryoid body (Example 6), human hepatocyte and a size marker. In the hepatocyte induced in Examples 5 and 6, the expression of albumin gene being gene specific to the hepatocyte is confirmed, but in particular, it can be confirmed that the expression is reinforced in the hepatocyte induced by three-dimensional culture using a non-woven cloth (Example 6). The result supports strongly that the differentiating induction to the hepatocyte can be more efficiently induced by using the non-woven cloth.

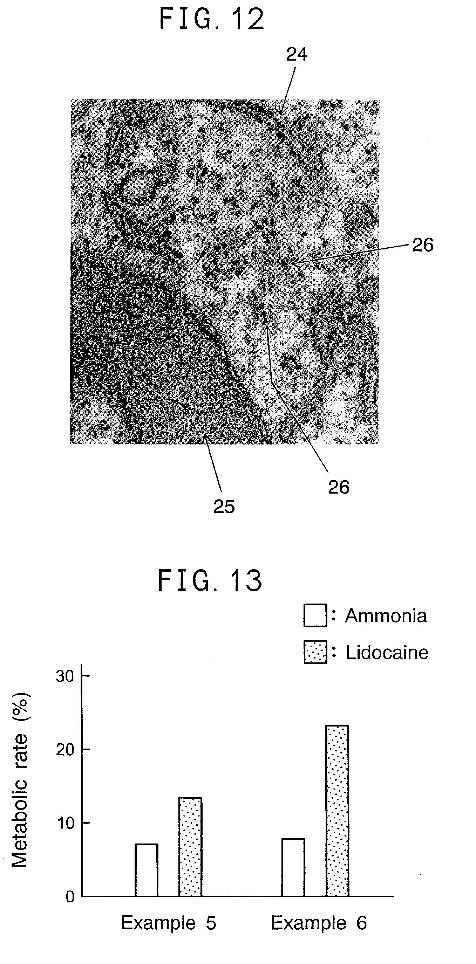

Further, glycogen granules characteristic for the grown-up hepatocyte (FIG.12) were observed in the cells of the human ES cell induced using a non-woven cloth by a transmission electric microscope. In FIG.12, the codes 24, 25 and 26 show respectively endoplasmic reticula, mitochondria and glycogen granules.

### TEST EXAMPLE 4

### Measurement of hepatic function

### (A) Metabolism of ammonia

The culture solutions of the hepatocytes obtained in Examples 5 to 6 were replaced with new culture solution after 14 days from the start of the differentiating induction. In the culture solutions, 1.4 mmol of ammonium tetrachloride was loaded, 10 µl of the culture solution was sampled after 4 hrs and the concentration of ammonia was measured. The metabolic rates of ammonia in Examples 5 to 6 are respectively 7.1 % and 7.8 %.

### (B) Metabolism of lidocaine

The culture solutions of the hepatocytes obtained in Examples 5 to 6 were replaced with new culture solution after 14 days from the start of the differentiating induction. In the culture solutions, lidocaine was loaded so as to be a final concentration of 1 mg/ml, 1000 µl of the medium was sampled after 4 hrs and the concentration of d lidocaine was measured. The metabolic rates of lidocaine in Examples 5 to 6 are respectively 13.4 % and 23.2 %.

The metabolic rate of each of the fore-mentioned (A) and (B) is shown in FIG.13. The metabolic rate is represented by dividing difference obtained by subtracting the concentration of each of drugs after 4 hrs from the concentration of a drug at loading the drug, by the concentration of the drug at loading the drug. Drug metabolic ability is observed in each of Examples, but it is grasped that the drug metabolic ability is enhanced by three-dimensional culture on a non-woven cloth.

### (D) Production of urea

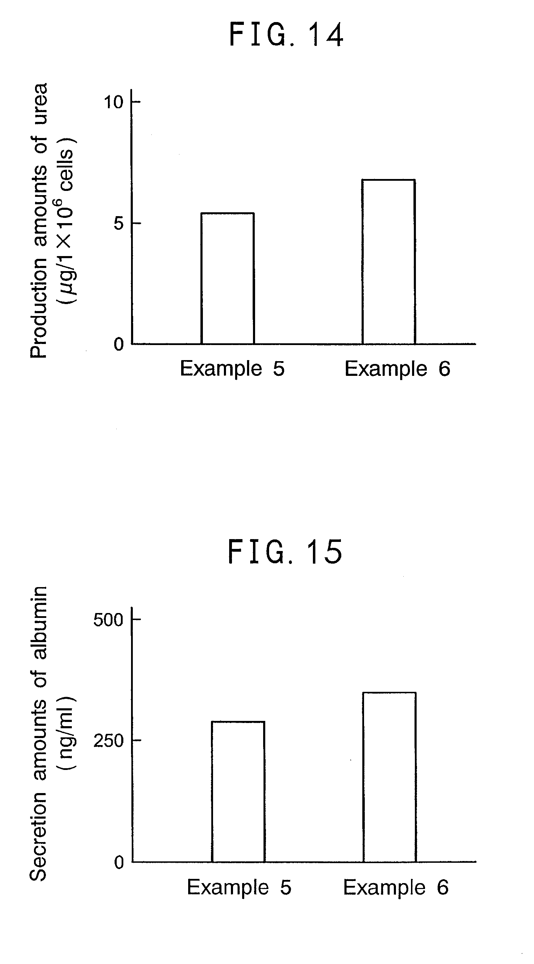

1.4 mmol of ammonium tetrachloride was loaded in like manner as the fore-mentioned (A), the culture solution was sampled after 4 hrs and the production amount of urea was measured. The production amounts were respectively 5.4 µg and 6.8 µg for Examples 5 to 6 (FIG. 14).

### (E) Production of albumin

The culture solutions of the hepatocytes obtained in Examples 5 to 6 were replaced with new culture solution after 14 days from the start of the differentiating induction. Then, the culture solutions after culturing for 24 hours were sampled and the production amount of albumin was measured using an albumin ELISA kit ALBUWELL II (Exocell Inc., Philadelphia, Pennsylvania State, USA). The production amounts of albumin were respectively 289 ng and 349 ng per 1 mlfor Examples 5 to 6 (FIG.15). It is grasped that the albumin production ability is enhanced by culture on a non-woven cloth and further by three-dimensional culture in the reactor.

### INDUSTRIAL APPLICABILITY

According to the differentially inducing method of the present invention, the safe hepatocyte that is adequately functionable for replacing the healthy human hepatocyte and able to supply in large quantity can be induced. Further, when the bioartificial liver of the present invention is made as an external type, it prevents dangerousness accompanied with cell migration and safer therapy can be realized. Further, in the present invention, a method of testing a drug using the human hepatocyte capable of being supplied in large quantity and a method for producing a physiologically active substance are provided.

### SEQUENCE LISTING FREE TEXT

SEQ ID No. 1: 5' primer for polymerase chain reaction to detect mouse CK 18 gene
SEQ ID No. 2: 3' primer for polymerase chain reaction to detect mouse CK 18 gene
SEQ ID No. 3: 5' primer for polymerase chain reaction to detect mouse G6P gene
SEQ ID No. 4: 3' primer for polymerase chain reaction to detect mouse G6P gene
SEQ ID No. 5: 5' primer for polymerase chain reaction to detect mouse TAT gene
SEQ ID No. 6: 3' primer for polymerase chain reaction to detect mouse TAT gene
SEQ ID No. 7: 5' primer for polymerase chain reaction to detect mouse albumin gene
SEQ ID No. 8: 3' primer for polymerase chain reaction to detect mouse albumin gene
SEQ ID No. 9: 5'primer for polymerase chain reaction to detect mouse AFP gene
SEQ ID No. 10: 3'primer for polymerase chain reaction to detect mouse AFP gene
SEQ ID No. 11: 5'primer for polymerase chain reaction to detect mouse HNF3b gene
SEQ ID No. 12: 3'primer for polymerase chain reaction to detect mouse HNF3b gene
SEQ ID No. 13: 5'primer for polymerase chain reaction to detect mouse GAPDH gene
SEQ ID No. 14: 3'primer for polymerase chain reaction to detect mouse GAPDH gene
SEQ ID No. 15: 5'primer for polymerase chain reaction to detect human albumin gene
SEQ ID No. 16: 3'primer for polymerase chain reaction to detect human albumin gene
SEQ ID No. 17: 5'primer for polymerase chain reaction to detect human β-actin
SEQ ID No. 18: 3'primer for polymerase chain reaction to detect human β-actin

## Claims

1. A method for differentially inducing embryo-stem cells into hepatocytes, wherein the embryo-stem cells are cultured in the presence of deletion type hepatocyte growth factor.

2. A method for differentially inducing embryo-stem cells into hepatocytes comprising the steps of:
(a) forming an embryoid body of the embryo-stem cells; and
(b) culturing the obtained embryoid body in the presence of deletion type hepatocyte growth factor.

3. The method of Claim 1 or 2, wherein the embryonic hepatocytes are derived from mammal.

4. The method of Claim 3, wherein the mammal is human.

5. The method of Claim 3, wherein the mammal is mouse.

6. The method of any one of Claims 2 to 5, wherein the embryoid body is formed by suspension culture.

7. The method of any one of Claims 1 to 5, wherein the culture is carried out by three-dimensional culture.

8. Hepatocytes induced by the method of any one of Claims 1 to 7.

9. A bioartificial liver comprising hepatocytes of Claim 8.

10. A process for producing the bioartificial liver comprising the steps of:
(a) forming an embryoid body of the embryo-stem cells;
(b) filling the obtained embryoid body in the bioartificial liver reactor; and
(c) culturing the embryoid body in the presence of deletion type hepatocyte growth factor in the bioartificial liver reactor.

11. A method for testing a drug using the hepatocytes of Claim 8.

12. A process for producing a physiologically active substance using hepatocytes of Claim 8.
